(19) Europäisches Patentamt
European Patent Office
Office européen des brevets

(11) **EP 2 845 899 B1**

(12) **EUROPEAN PATENT SPECIFICATION**

(45) Date of publication and mention
of the grant of the patent:
**31.10.2018  Bulletin 2018/44**

(51) Int Cl.:
**C12N 5/0793** (2010.01)

(21) Application number: **14179316.6**

(22) Date of filing: **31.07.2014**

(54) **METHOD FOR PREPARING DOPAMINERGIC NEURON-LIKE CELL CLUSTER**

VERFAHREN ZUR HERSTELLUNG VON DOPAMINERGEN NEURONEN ÄHNLICHER ZELLHAUFEN

PROCÉDÉ DE FABRICATION D'UN AGRÉGAT DE CELLULES DE TYPE NEURONES DOPAMINERGIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **02.08.2013  KR 20130091966**

(43) Date of publication of application:
**11.03.2015  Bulletin 2015/11**

(73) Proprietor: **Daegu Gyeongbuk Institute of Science and Technology**
**Daegu 711-873 (KR)**

(72) Inventors:
• **Jeon, Won Bae**
**711-873 Daegu (KR)**
• **Lee, Kyeong Min**
**704-770 Daegu (KR)**
• **Jung, Gwon Soo**
**704-770 Daegu (KR)**

(74) Representative: **Fuchs Patentanwälte Partnerschaft mbB**
**Westhafenplatz 1**
**60327 Frankfurt am Main (DE)**

(56) References cited:
KR-A- 20130 047 404

• GWON-SOO JUNG ET AL: "Morphogenetic and neuronal characterization of human neuroblastoma multicellular spheroids cultured under undifferentiated and all-trans-retinoic acid-differentiated conditions", BMB REPORTS, vol. 46, no. 5, 31 May 2013 (2013-05-31), pages 276-281, XP055164693, ISSN: 1976-6696, DOI: 10.5483/BMBRep.2013.46.5.196

• S. SHAVALI ET AL: "Synergistic Neurotoxic Effects of Arsenic and Dopamine in Human Dopaminergic Neuroblastoma SH-SY5Y Cells", TOXICOLOGICAL SCIENCES, vol. 102, no. 2, 18 October 2007 (2007-10-18), pages 254-261, XP055164714, ISSN: 1096-6080, DOI: 10.1093/toxsci/kfm302

• WON BAE JEON ET AL: "Functional enhancement of neuronal cell behaviors and differentiation by elastin-mimetic recombinant protein presenting Arg-Gly-Asp peptides", BMC BIOTECHNOLOGY, BIOMED CENTRAL LTD. LONDON, GB, vol. 12, no. 1, 14 September 2012 (2012-09-14), page 61, XP021121077, ISSN: 1472-6750, DOI: 10.1186/1472-6750-12-61

• GILDA RAGUENEZ ET AL: "BCL-2 Is Upregulated in Human SH-SY5Y Neuroblastoma Cells Differentiated by Overexpression of Fibroblast Growth Factor 1", BIOCHEMICAL AND BIOPHYSICAL RESEARCH COMMUNICATIONS, vol. 258, no. 3, 1 May 1999 (1999-05-01), pages 745-751, XP055164803, ISSN: 0006-291X, DOI: 10.1006/bbrc.1999.0613

EP 2 845 899 B1

- Elena Di Daniel ET AL: "Comparative analysis of the effects of four mood stabilizers in SH-SY5Y cells and in primary neurons", , 1 February 2005 (2005-02-01), XP055164813, Retrieved from the Internet: URL:http://onlinelibrary.wiley.com/doi/10.1111/j.1399-5618.2004.00164.x/pdf [retrieved on 2015-01-26]
- KUME T ET AL: "Dibutyryl cyclic AMP induces differentiation of human neuroblastoma SH-SY5Y cells into a noradrenergic phenotype", NEUROSCIENCE LETTERS, LIMERICK, IE, vol. 443, no. 3, 10 October 2008 (2008-10-10), pages 199-203, XP024523549, ISSN: 0304-3940, DOI: 10.1016/J.NEULET.2008.07.079 [retrieved on 2008-08-05]
- BLACK S A G ET AL: "Rapid, transient effects of the protein kinase C activator phorbol 12-myristate 13-acetate on activity and trafficking of the rat high-affinity choline transporter", NEUROSCIENCE, NEW YORK, NY, US, vol. 167, no. 3, 19 May 2010 (2010-05-19), pages 765-773, XP026997491, ISSN: 0306-4522 [retrieved on 2010-02-16]

**Description**

BACKGROUND OF THE INVENTION

1. Field of the Invention

The present invention relates to a method for preparing a dopaminergic neuron-like cell cluster. More particularly, the present invention relates to a method for preparing a dopaminergic neuron-like cell cluster exhibiting a far higher level of a dopaminergic neuron marker, compared to conventional 2D or 3D cultured cells.

2. Description of the Related Art

Cell culture is the complex process by which cells isolated from a donor organism are separated into single cells through treatment with, for example, proteinases, and are grown under controlled conditions. Culturing of animal cells, also known as tissue culture, started from the early 1900s. At that time, however, tissue culture was performed by aseptically selecting a biotissue, finely segmenting the biotissue with scissors or a razor, growing or culturing the tissue segments to give cell clusters, and scraping cell clusters from the test tube wall to achieve phase separation or cell passage,
; however it did not involve the culturing of single cells. It was in the 1950s that modern-day cell culture started, with the development of trypsinization-based cell dispersion. Cell culture technology now makes it possible to treat cells as single-celled organisms. Based on the knowledge or information obtained from E. coli, etc. through cell culture technology, there has been established a methodology of analyzing animal cells. As a result, various biological mechanisms including metabolism, proliferation, differentiation, aging, infection with oncogenic viruses, and the like can be quantitatively analyzed at a cellular level.

There are two basic systems for growing cells in culture, as monolayers adherent on an artificial substrate, so-called adherent culture; or free-floating in a culture medium, so-called suspension culture. In addition, individual cells may be grown to form colonies on plates, or to do so in the form of a large mass scale within a liquid medium. Cultured cells have morphologies resembling those from which they are sourced, such as: epithelium-like, fibroblast-like, lymphocyte-like morphologies. Primarily cultured cells, that is, the first cell line resulting after separation from somatic cells, are of normal diploidy. Additionally, primarily cultured cells have normal properties, but may undergo changes in proliferative ability, chromosome number, morphologies, etc. (transformation), and may become oncogenic, with an increase in passage. Sometimes, they may lose the ability to differentiate into the tissues from which they are derived. Recently, various undifferentiated cell lines that can differentiate into specialized cells have been established.

This cell culture technology is one of the most important technologies supporting modern-day bioengineering. Without the cell culture technology, it is impossible either to produce recombinant or hybrid cells having foreign DNA to produce desired matter or monoclonal antibodies, or to breed plant cells with new properties.

Among the fields in which cell culture finds its applications is the development of medications. Drug development includes pre-clinical research (using microorganisms/animals) and clinical trials (on humans) for verifying the effectiveness of a drug candidate in the diagnosis and treatment of a disease, and the safety of the drug candidate for humans; and may include the step of submitting an application form on which the trial results are summarized for the purpose of obtaining regulatory approval to market the drug. In pre-clinical research, experiments are conducted with animals. Animal experiments require a great expense, and cause controversial ethical issues. Thus, an alternative to animal experiments is a social and moral necessity, and must be achieved in terms of expense reduction for companies. Cell culture on a mass production scale is highly regarded as a solution to the problem with animal experiments, and has been actively studied.

In addition, in vitro assay using cells is regarded as having an advantage over conventional animal experiments in elucidating the pharmacokinetic mechanisms of drugs or compounds, which is difficult to achieve through animal experiments.

The organs of the nervous system, such as the brain, are comprised of numerous and diverse cells that are different in function and property, like neurons (nerve cells) and glial cells. Dysfunction or death of nerve cells, so-called neuronal degeneration, occurs due to various causes, resulting in significant diseases within the body. Representative examples of neurodegenerative diseases caused by the death of specific nerve cells include: Alzheimer's disease, Parkinson's disease and Huntington's disease. Particularly, research into the onset mechanism and treatment of Parkinson's disease may be more effectively carried out by an in vitro assay using cells.

Accordingly, the three-dimensional cell cluster culture of various cells, including primary cells, stem cells, and tumor cells; has recently been attempted. This is because cells that are difficult to culture, like induced pluripotent stem cells and nervous stem cells, should be cultured in a large quantity in an early stage. To this end, extensive studies have been conducted particularly towards the use of substrates, such as an extracellular matrix isolated directly from animal tissues, or synthetic organic polymers, in culturing three-dimensional cell clusters.

However, the extracellular matrix (ECM) for use in culturing three-dimensional cell clusters, directly isolated from animal tissues, is structurally complicated, restrictively produced, and highly costly. The likelihood of potential contamination with unidentified pathogens acts as a barrier against promising pertinent reliability for biological safety. Further, naturally derived ECM may include mutations which may lead to an unexpected and invalid results.

In lieu of the ECM directly isolated from animal tissues, organic polymers such as polyethylene glycol may be used as substrates to overcome the problems with ECM. When applied to cell culture, organic polymers may cause multiple mutations owing to their intrinsic polydispersity, and arouse the cellular toxicity of the innate immune system against the chemical substrate.

When general media are employed, cells are not cultured in a three-dimensional pattern, but in a two-dimensional pattern from which it is difficult to understand the in-vivo action mechanism of neurons because of discrepancy between in-vivo three-dimensional arrangements and two-dimensional arrangements of neurons.

In addition, although undifferentiated cells are cultured in the above-mentioned manner to form 3D cell clusters, cells pertinent to the goal of study on neurons, such as treatment of Parkinson's disease are difficult to acquire. Even in the presence of a separate differentiation-inducing factor, neuroblast cells that are cultured in the conventional two- or three-dimensional manner are poorly induced to differentiate into dopaminergic neurons suitable for use in studying neurodegenerative diseases; in addition to encountering the problem of additionally separating from the differentiated dopaminergic neurons.

In Korean Patent Application No. 2011-0112406, the present inventors disclosed a method for forming 3-dimensional multicellular spheroids using an artificial elastin-like extracellular matrix, and 3D multicellular spheroids established thereby. In light of the technical level at the time of the application, the 3D multicellular spheroids established by the method were, however, anticipated to poorly differentiate into dopaminergic neurons even in the presence of a dopaminergic neuron differentiation-inducing factor, as in conventional techniques.

In addition, nowhere is the fact that the 3D cell culture cluster cultured in the method of the application is induced in the presence of a differentiation-inducing factor to differentiate into dopaminergic neurons at very high rates suggested or emphasized in previous documents. Hence, the technical level at the time of the application cannot guide the formation of 3D dopaminergic neuron clusters, which are high enough in numbers, towards effectively studying neurodegenerative diseases.

"Morphogenetic and neuronal characterization of human neuroblastoma multicellular spheroids cultured under undifferentiated and all-trans-retinoic acid-differentiated conditions (Gwon-Soo Jung et al., BMB Reports 46(5) p. 276 - 281, 31 May 2013)" discloses a method wherein the neuroblastoma cell line SH-SY5Y has been grown on a dish coated with an elastin-like polypeptide having the formula $TGPG[VGRGD(VGVPG)_6]_{20}WPC$, named RGD-ELP, spheroids were formed, and neurite outgrowth was observed under retinoic acid-induced differentiation conditions.

The article "Synergistic neurotoxic effects of arsenic and dopamine in human dopaminergic neuroblastoma SH-SY5Y Cells" from S. Shavali et al. (Toxicological Sciences 102(2), p. 254 - 261, 18 October 2007) describes the use of the SH-SY5Y neuroblastoma cell line, which possesses differentiated characteristics of dopaminergic neurons to study the degeneration of dopaminergic neuronal cells as a model for Parkinson's disease. The SH-SY5Y cell line was cultured in Dulbecco's modified Eagle's medium (DMEM) and was treated with Arsenic, Dopamin, Dopamin + Arsenic, Dopamin transporter blockers and Dopamin antagnosists.

Won Bae Jeon et al. disclose in their arcticle "Functional enhancement of neuronal cell behaviors and differentiation by elastin-mimetic recombinant protein presenting Arg-Gly-Asp peptides" (BMC Biotechnology 12(1) p. 61, 14 September 2012) a method for forming three-dimensional cell aggregate using an elastine-like artificial extracellular substrate having the structure VGRGD(VGVPG) and using the neuroblastoma cell line N2a, differentiating by retinoic acid. This method is also described in KR 2013 0047404 A.

In "BCL-2 is upregulated in human SH-SY5Y neuroblastoma cells differentiated by overexpression of fibrolast growth factor 1" from Gilda Raguenez et al. (Biochemical and biophysical research communications 258(3) p. 745 - 751, 1 May 1999) the authors mention that the treatment of SH-SY5Y cells with phorbol esters, retinoic acid, staurosporine, fibroblast growth factor 2 or epidermal growth factor induces cell differentiation. They use the SH-SY5Y cell line to study cell differentiation induced by fibroblast growth factor 1.

In the article "Comparative analysis of the effects of four mood stabilizers in SH-SY5Y cells and in primary neurons" from Elena Di Daniel et al. (Bipolar Disorders 7(1), p. 33 - 41 1 February 2005) the scientists used, amongst others, phorbol 12-myristate 13-acetate and epidermal growth factor for the differentiation of the neuroblastoma cell line SH-SY5Y.

Kume et al. disclose in their article "Dibutyryl cyclic AMP induces differentiation of human neuroblastoma SH-SY5Y cells into a noradrenergic phenotype" (Neuroscience Letters 443(3) p. 199-203, 10 October 2008) a method to plate the SH-SY5Y cells on poly-L-lysine-coated cover glass differentiated by dibutyryl cyclic AMP or retinoic acid.

SUMMARY OF THE INVENTION

Accordingly, the present invention has been made keeping in mind the above problems occurring in the prior art, and an object of the present invention is to provide a method for preparing a 3D dopaminergic neuron-like cell cluster which can be more effectively differentiated into dopaminergic neurons in the presence of a differentiation-inducing factor, compared to that prepared by conventional 2D or 3D cell culture methods, so that it is suitable for use in studying neurodegenerative diseases such as Parkinson's disease.

Another object of the present invention is to provide a method for preparing a 3D dopaminergic neuron-like cell cluster by which factors reinforcing intercellular interaction can be expressed at an improved level from the 3D dopaminergic neuron-like cell cluster.

A further object of the present invention is to provide a method for preparing a 3D dopaminergic neuron-like cell cluster which is: free of cellular toxicity and safe to the body, easy to culture with high productivity, superior in biocompatibility, and highly unlikely to undergo mutations.

A still further object of the present invention is to provide a dish for culturing a 3D dopaminergic neuron-like cell cluster on a mass scale; which guarantees the 3D dopaminergic neuron-like cell cluster superior biocompatibility, freedom of cellular toxicity, minimal mutagenicity, and improvement in the expression of factors reinforcing intercellular interaction.

In order to achieve the first three objects thereof, the present invention provides a method for preparing a 3D dopaminergic neuron-like cell cluster, comprising coating a cell culture dish with an artificial elastin-like extracellular matrix having the amino acid set forth in the following General Formula 1:

$$[(N\text{-terminus})\text{-TGPG}[\text{VGRGD}(\text{VGVPG})_\alpha]_\beta\text{WPC-}(C\text{-terminus})]$$

wherein $\alpha$ is an integer meeting $2\leq \alpha \leq 10$, and $\beta$ is an integer meeting $10\leq \beta \leq 30$.
; culturing neuroblast cells in the coated cell culture dish to form a 3D neuroblast cell cluster; and adding a dopaminergic neuron differentiation-inducing factor to the 3D neuroblast cell cluster to form a 3D dopaminergic neuron-like cell cluster.

In another preferred embodiment of the present invention, the coating may be carried out by applying the artificial elastin-like extracellular matrix in an amount of 0.05 to 100 $\mu$mol per surface area (mm$^2$) of the culture dish.

In another preferred embodiment of the present invention, the neuroblast is selected from the group consisting of: N2a, SH-SY5Y, and a combination thereof.

In the present invention, the dopaminergic neuron differentiation-inducing factor is phorbol 12-myristate 13-acetate. In a preferred embodiment of the present invention, the adding may be carried out by using the differentiation-inducing factor in an amount of $1\times10^{-7}$ to $1\times10^{-2}$ parts by weight, based on 100 parts by weight of the 3D neuroblast cell cluster.

In order to achieve the fourth object thereof, the present invention provides a dish for culturing dopaminergic neuron-like cell clusters comprising an artificial elastin-like extracellular matrix coating the dish to enhance intercellular interaction, the artificial elastin-like extracellular matrix having the amino acid set forth in the following General Formula 1:

$$[(N\text{-terminus})\text{-TGPG}[\text{VGRGD}(\text{VGVPG})_\alpha]_\beta\text{WPC-}(C\text{-terminus})]$$

wherein $\alpha$ is an integer meeting $2\leq \alpha \leq 10$, and $\beta$ is an integer meeting $10\leq \beta \leq 30$.

The amino acid sequence used in the present invention is expressed with abbreviations for amino acids according to the IUPAC-IUB nomenclature, as listed in Table 1.

TABLE 1

| IUPAC-IUB Name | One-Letter Notation | IUPAC-IUB Name | One-Letter Notation | IUPAC-IUB Name | One-Letter Notation |
|---|---|---|---|---|---|
| Alanine | A | Glycine | G | Proline | P |
| Arginine | R | Histidine | H | Serine | S |
| Asparagine | N | Isoleucine | I | Threonine | T |
| Aspartic acid | D | Leucine | L | Tryptopha n | w |
| Cysteine | c | Lysine | K | Tyrosine | Y |

(continued)

| IUPAC-IUB Name | One-Letter Notation | IUPAC-IUB Name | One-Letter Notation | IUPAC-IUB Name | One-Letter Notation |
|---|---|---|---|---|---|
| Glutamic acid | E | Methionine | M | Valine | V |
| Glutamine | Q | Phenylalani ne | F | | |

Prior to the elucidation of the present invention, terms used herein are defined as follows.

As used herein, the term "extracellular matrix (ECM)" refers to an intercellular part surrounding cells, and having a network structure composed mainly of proteins and polysaccharides.

As used herein, the term "artificial extracellular matrix" refers to an extracellular matrix, which plays an important role in cell attachment, migration and differentiation, artificially prepared by means of, for example, gene cloning; and is intended to encompass an artificial protein containing the arginyl glycyl aspartic acid (Arg-Gly-Asp (RGD)) motif involved in integrin mediated interaction, with an elastin mimetic protein serving as a backbone.

In addition, the term "3-dimensional cell cluster" means an aggregation of cells with a three-dimensional pattern, which is artificially constructed to resemble a bio-tissue by inducing cell-to-cell interaction based on the expression of gap junction proteins, such as cadherin and connexin. Whereas a 2D cell cluster has a monolayer of cells, a 3D cell cluster has multilayered cells. Hence, 3D cell clusters are higher in cell density per unit area than are 2D cell clusters.

The term "spheroid" refers to a 3-dimensional oval morphology of a cell aggregate, and the 3D cell cluster of the present invention has a spheroidal morphology.

The term "blast cell," as used herein, refers to a cell that is undifferentiated or differentiated only up to several times, and that is rich in RNA, with the onset of vigorous DNA synthesis. In addition, "blast cell" is intended to encompass a cell that synthesizes and secretes collagenous fibers, like a fibroblast.

As used herein, the term "SH-SY5Y cells" refers to an established cell line derived from the cell line SK-N-SH originating from a bone marrow biopsy taken from a person with neuroblastoma.

The term "established cell line" means a cell line, derived from a multicellular organism, which has acquired the ability to proliferate indefinitely (subculture)

The term "differentiation-inducing factor," as used herein, is intended to encompass any protein or compound that functions to induce the maturation and differentiation of undifferentiated neuroblasts.

By the term "dopaminergic neuron" is meant the neuron that synthesizes dopamine and releases it as a neurotransmitter. Dopaminergic neurons are distributed in the midbrain reticular formation, the substantia nigra, and the ventral tegmental area, projecting to the striatum and the limbic system. In the biosynthesis pathway of dopamine, the reaction catalyzed by tyrosine hydroxylase is a rate determining step. Parkinson's disease results from the degeneration of dopaminergic neurons in the nigrostriatal pathway.

"Tyrosine hydroxylase (TH)" is the enzyme responsible for the conversion of the amino acid L-tyrosine to 3,4-dihydroxyphenylalanine (L-DOPA) by using a molecular oxygen to hydroxylate its substrate. Tyrosine hydroxylase catalyzes the rate limiting step in this synthesis of catecholamines, and is present in regions where the biosynthesis of catecholamines actively occurs. The enzyme is a tetramer of four identical subunits, each about 5.6 kDa (homotetramer), and has non-heme iron as a central element of the active site.

"Quantitative real time PCR (qRT-PCR)" is a laboratory technique of molecular biology based on the polymerase chain reaction (PCR) in which complementary DNA (cDNA) reversely transcribed from RNA by a reverse transcriptase serves as a template to amplify and simultaneously detect or quantify a targeted DNA molecule with the aid of target probes comprising target primers and a marker.

"A primer" is a strand of nucleic acid having a free 3' end that can form base pairs with a complementary template and serves as a starting point for the replication of the template. Given primers together with a suitable buffer and reagents (that is, DNA polymerase or reverse transcriptase, four different nucleoside triphosphates, etc.), PCR can be performed at predetermined temperatures. Additional features may be incorporated into primers so long as the primer's fundamental function of acting as a starting point is not altered by the features.

Three-dimensional cell clusters which are cultured but remain undifferentiated are difficult to apply for studying neurodegenerative diseases, such as Parkinson's disease. In addition, two-dimensionally cultured nerve cells are insufficient as a model available for the study of mechanisms occurring in the in-vivo 3D nerve cell entity. Further, cells grown by conventional 2D- or 3D-cell culture techniques are very poor in differentiation rate even in the presence of a differentiation-inducing factor. Designed to overcome the problems encountered in the conventional techniques, the present invention provides a method for preparing a 3D dopaminergic neuron-like cell cluster which is highly apt to differentiate into dopaminergic neurons so that it can be effectively applied for studying neurodegenerative diseases, such as Parkinson's disease, without an additional process of isolating dopaminergic neurons.

Over conventional techniques, in addition, the present invention has the advantage of improving the expression of factors responsible for intercellular interaction, and producing 3D cell clusters in a large quantity only by simple coating with a culture matrix, and highly biocompatible 3D dopaminergic neuron-like cell clusters by employing an elastin-like matrix causative of no rejection reactions with living cells.

Moreover, the present invention gives no biological hazards attributed to potential contamination because there is no likelihood that unidentified pathogens would remain in the matrix. Also, the biomimetic matrix used in the present invention does not arouse the cellular toxicity of the innate immunity system.

BRIEF DESCRIPTION OF THE DRAWINGS

The above and other objects, features and other advantages of the present invention will be more clearly understood from the following detailed description taken in conjunction with the accompanying drawings, in which:

FIG. 1 is a graph showing mRNA levels of CAMs in the 3D cell cluster according to one preferred embodiment of the present invention;
FIG. 2 is a graph showing mRNA levels of ECM components in the 3D cell clusters according to one preferred embodiment of the present invention;
FIG. 3 shows microphotograph images of neuroblast cells cultured according to one preferred embodiment of the present invention;
FIG. 4 shows SEM images of cells cultured according to a preferred embodiment of the present invention and a comparative embodiment;
FIG. 5 shows microphotograph images of 3D neuroblast cell clusters according to one preferred embodiment of the present invention after a viability assay;
FIG. 6 is a graph in which the 3D dopaminergic neuron-like cell cluster according to one preferred embodiment of the present invention is compared with 2D dopaminergic neuron-like cells in terms of tyrosine hydroxylase (TH) activity; and
FIG. 7 shows relative tyrosine hydroxylase activity of the cells differentiated from 3D cells clusters cultured by a conventional method or the method of the present invention.

DESCRIPTION OF THE PREFERRED EMBODIMENTS

Below, a detailed description will be given of the present invention, with reference to the accompanying drawing.

As mentioned above, conventional techniques are problematic in that 3D cell clusters which remain undifferentiated are difficult to apply for studying on neurodegenerative diseases, such as Parkinson's disease, in that two-dimensionally cultured nerve cells are insufficient as a model available for the study of mechanisms occurring in the in-vivo 3D nerve cell entity, and in that cells grown by conventional 2D- or 3D-cell culture techniques are very poorly differentiated even in the presence of a differentiation-inducing factor, and require an additional process of separating the differentiated dopaminergic neurons.

For use in culturing 3D cell clusters, extracellular matrices directly extracted from animal tissues are restrictively produced, and expensive, and cannot promise pertinent reliability to biological safety. Further, they may retain a mutation, which leads to an unexpected, invalid result.

Organic polymers, such as polyethylene glycol, may be used instead of the ECM directly extracted from animal tissues, but may cause multiple mutations owing to their intrinsic polydispersity, and arouse the cellular toxicity of the innate immune system against the chemical substrate.

In accordance with an aspect thereof, the present invention addresses a method, as a solution to the above-mentioned problems, for preparing a 3D dopaminergic neuron-like cell cluster, comprising coating a cell culture dish with an artificial elastin-like extracellular matrix configured to reinforce intercellular interaction having the amino acid set forth in the following General Formula 1:

$$[(\text{N-terminus})\text{-TGPG}[\text{VGRGD}(\text{VGVPG})_\alpha]_\beta\text{WPC-}(\text{C-terminus})]$$

wherein $\alpha$ is an integer meeting $2 \leq \alpha \leq 10$, and $\beta$ is an integer meeting $10 \leq \beta \leq 30$
; culturing neuroblast cells in the coated cell culture dish to form a 3D neuroblast cell cluster; and adding a dopaminergic neuron differentiation-inducing factor to the 3D neuroblast cell cluster to form a 3D dopaminergic neuron-like cell cluster.

The method of the present invention can produce dopaminergic neuron-like cell clusters in large quantities as well as guarantee an excellent rate of differentiation into dopaminergic neurons, compared to 2D or 3D cell culture techniques. In addition, the dopaminergic neuron-like cell cluster prepared according to the method is highly biocompatible and biologically safe, and does not exert cellular toxicity in the innate immune system because of the use of a biomimetic substrate in the preparation.

For use in the preparation of 3D dopaminergic neuron-like cell clusters, the cell culture dish is coated with an artificial elastin-like extracellular matrix in accordance with the method of the present invention.

In the context of the use of the artificial elastin-like extracellular matrix, a description is first given of the in-vivo extracellular matrix and its role. The ECM is a complex collection of specialized biopolymers proteins secreted by cells that provides structural and biochemical support to the surrounding cells. The ECM is composed of fibrous proteins such as collagens, elastin, etc., complex proteins such as proteoglycans, glycosaminoglycans, etc., and cell adhesion glycoproteins, such as fibronectin, laminin, vitronectin, etc. The ECM takes charge of physical functions including support and attachment to the cells, physical boundary formation, absorption of mechanical strains applied to tissues, and provision of elastic elements while biologically functioning to control cell proliferation, cell migration, intracellular metabolism, cell differentiation, and morphology outside the cells. Without the structure properties of the ECM, it would be impossible for cells to maintain certain morphologies during division and to bind to each other.

Mimicking the ECM, the artificial elastin-like extracellular matrix functions to improve cell-to-cell interaction so that the dividing cells bind to each other and thus are not cultured in a monolayer form, but grown to form 3D multicellular spheroids.

In detail, when cultured in a typical culture medium, cells are more likely to adhere to the culture medium than adjacent cells, thus growing in a two-dimensional form. Cells in a two-dimensional configuration on a culture medium are different from in-vivo cells which are three-dimensionally connected with each other, so that they are unsuitable for use in studying cellular mechanisms.

In the present invention, the artificial elastin-like extracellular matrix on a culture dish functions to induce signaling with receptors, such as integrin, on the membrane of the cells, thereby increasing intercellular interactions of the cells cultured in the dish. In this condition, the cells bind to each other, forming a spheroidal 3D cell cluster.

The RGD (Arginyl Glycyl Aspartic acid, Arg-Gly-Asp) motif is involved in the cell signaling pathway between the ECM and the membrane receptor integrin, serving as a binding site which the integrin receptor recognizes, and is found in various proteins such as fibronectin, hydronectin, laminin, collagen, etc.

When binding to the integrin receptor of cells, the RGD motif stimulates signaling pathways within the cultured cells, increasing the expression of cell adhesion molecular (hereinafter referred to as "CAMs") such as N-cadherin, neural cell adhesion molecule-2 (hereinafter referred to as NCAM2), intercellular cell adhesion molecule-1 (hereinafter referred to as "ICAM1") and connexin- 26, and thus encouraging the formation of 3D cell clusters.

An increase in the expression of CAMs and ECM improves cell-to-cell interaction, which may lead to facilitating the formation of 3D cell clusters.

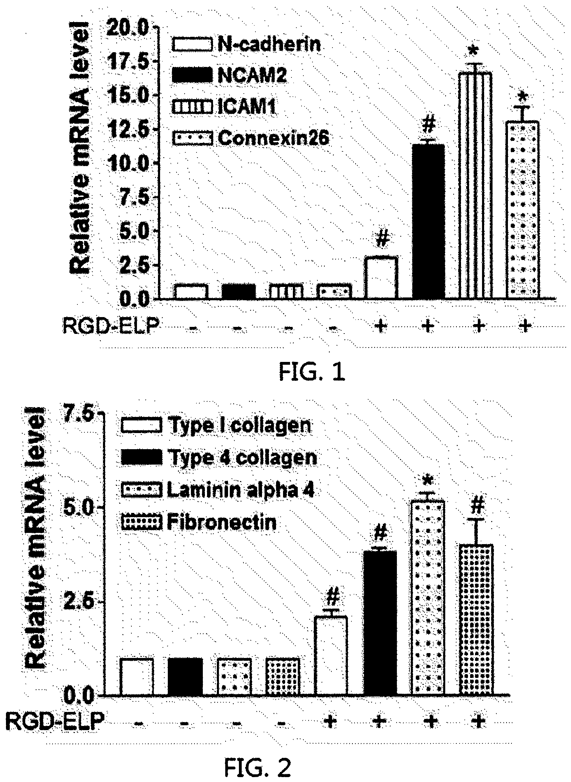

With reference to FIG. 1, mRNA levels of CAMs in the 3D cell cluster according to one preferred embodiment of the present invention are depicted. In the presence of the artificial elastin-like extracellular matrix (RGD-ELP), N-cadherin, NCAM2, ICAM1, and connexin-26 were observed to increase in their respective mRNA levels, compared to the absence of RGD-ELP. Upon translation, their protein levels may be elevated in proportion to the respective increased mRNA levels.

FIG. 2 is a graph showing mRNA levels of ECM components in the 3D cell clusters according to one preferred embodiment of the present invention. As shown, the cells cultured in the presence of the artificial elastin-like extracellular matrix (RGD-ELP) increased in mRNA levels of the ECM proteins including collagens (type 1, type 4), laminin $\alpha 4$ and fibronectin. Upon translation, their protein levels may be elevated in proportion to the respective increased mRNA levels.

The artificial elastin-like extracellular matrix may comprise a protein having the amino acid set forth in the following General Formula 1:

$$[(N\text{-}terminus)\text{-}TGPG[VGRGD(VGVPG)_\alpha]_\beta WPC\text{-}(C\text{-}terminus)]$$

wherein $\alpha$ is an integer meeting $2\leq \alpha \leq 10$, and $\beta$ is an integer meeting $10\leq \beta \leq 30$.

The elastin-like extracellular matrix represented by General Formula I is composed largely of an elastin-like peptide and an RGD motif.

In the elastin-like extracellular matrix represented by General Formula I, the RGD motif stimulates the expression of CAMs and the ECM, as described above, thus encouraging the formation of 3D cell clusters. However, once neuroblast cells adhere to the culture dish, the formation of 3D cell clusters cannot be strongly driven.

To overcome this problem, an elastin-like peptide is conjugated with the RGD motif in the present invention. Composed essentially of the hydrophobic amino acids glycine (G), valine (V) and proline (P), the elastin-like peptide can make the surface of the culture dish hydrophobic when it is coated with the elastin-like extracellular matrix. In this condition, the cells are reluctant to adhere to the cell dish, but are subjected to the promotion of cell behaviors such as diffusion and migration and thus induced to aggregate together. In addition, the RGD motif can facilitate the formation of 3D clusters of neuroblast cells, in synergy with the elastin-like peptide conjugated therewith.

In addition, the elastin-like peptide is non-immunogenic enough to not cause a rejection response to viable cells, thus culturing cells in a biocompatible manner.

Meanwhile, in order that the elastin-like extracellular matrix in which the RGD motif is conjugated with the elastin-like peptide (ELP) effectively functions to induce and facilitate the formation of 3D cell clusters, the RGD motif should be exposed toward the outside of the matrix so that it can readily bind to the integrin receptor on the membrane of the cells. For this, the relative size between the RGD motif and the elastin-like peptide (ELP) is an important factor.

In General Formula I for the artificial elastin-like extracellular matrix, $\alpha$ is an integer set to be $2 \leq \alpha \leq 10$ and, $\beta$ is an integer set to be $10 \leq \beta \leq 30$. In one preferred embodiment, $\alpha$ is an integer of from 4 to 8, and $\beta$ is an integer of from 18 to 22.

If $\alpha$ and $\beta$ are outside the ranges, the formation of 3D cell clusters may be little proceeded. Particularly, when the RGD motif is small in size, compared to the elastin-like peptide in the artificial elastin-like extracellular matrix, there may occur steric hindrance or conformation change effects that restrict the RGD motif to approach surface receptors, such as integrin, on the membrane of the cultured cells, thus resulting in failure in the formation of 3D cell clusters.

As for the cell culture dish, its material may be selected from the group consisting of, but not limited to, polystyrene, stainless steel, glass, a biopolymer, and a combination thereof. So long as it is typically used in cell culture, any dish may be employed in the present invention.

Further, the cell culture vessel may be in the form of a schale, a test tube, or an Erlenmeyer flask. However, so long as it is used for typical cell culture, any vessel may be taken without limitations, and will further be referred to herein as the "cell culture dish" or simply "dish".

The cell culture dish may preferably have a volume of from 0.32 to 20 cm$^3$, and an artificial elastin-like extracellular matrix-coated area of from 0.32 to 20 cm$^2$. However, these numerical ranges are limitative examples, but may vary in consideration of the quantity of 3D cell clusters.

Next, a description turns to coating the cell culture dish with the artificial elastin-like extracellular matrix.

The coating process may be carried out by, but not limited to, thermally induced coacervation at 37°C using a coating solution of the artificial elastin-like extracellular matrix in a solvent. So long as it allows a cell culture dish to be coated with the artificial elastin-like extracellular matrix, any coating process may be used without limitations.

The coating thickness of the artificial elastin-like extracellular matrix is not particularly limited, but may preferably range from 0.001 to 10 pm.

Preferably, the coating solution may be prepared by mixing the artificial elastin-like extracellular matrix in an amount of 0.05 to 2 parts by weight, based on 100 parts by weight of a solvent. Examples of the solvent include, but are not limited to, phosphate buffered saline (PBS) and water, with preference for PBS. So long as it is typically applied for the coating of a cell culture dish without modifying the artificial elastin-like extracellular matrix, any solvent may be used without limitations.

The amount of the coating solution used may be determined according to the volume or surface of the cell culture dish. Preferably, the coating solution may be used in such an amount as to apply 0.05 to 100 $\mu$mol of the artificial elastin-like extracellular matrix per surface area (mm$^2$) of the cell culture dish. When the artificial elastin-like extracellular matrix is used in an amount less than 0.05 $\mu$mol per mm$^2$ of the cell culture dish, there may be a reduction in the binding of the matrix to the integrin receptor of the cells and thus in stimulating the signaling pathway which may, in turn, decreases cell aggregation, resulting in a failure in the formation of 3D cell clusters. On the other hand, when the concentration of the artificial elastin-like extracellular matrix exceeds 100 $\mu$mol, the cells may fail to form 3D cell clusters. This is because the cell-to-cell interaction induced by recognizing the amino acid order of the RGD motif on the matrix may be specifically inhibited when an excess of the RGD motif is present.

In a subsequent step, neuroblast cells are cultured in the coated cell culture dish to form a 3D neuroblast cell cluster.

As used herein, "neuroblast cell" is an undifferentiated cell which will develop into a neuron. The neuroblast cell is at least one selected from the group consisting of N2a, SH-SY5Y, and preferably may be SH-SY5Y.

The neuroblast cells may be seeded to the coated cell culture dish at a density of $1 \times 10^4$ to $3 \times 10^6$ cells/mm$^3$ and preferably at a density of $5 \times 10^4$ to $5 \times 10^5$ cells/mm$^3$. This range, however, does not act as a limitative factor, and the density at which the cells are seeded may vary within such a range that the cell culture is not weakened.

Culture conditions for the seeded neuroblast cells are as follows.

First, a typical medium for neuroblast cells may be used. Preferable is a 10% complete medium. The 10 % complete medium may be prepared by mixing DMEM at a volume ratio of 1 : 1 ~ 2 with Ham's F12 medium, and supplementing the mixture with 3~10 vol% of fetal bovine serum, 50 ~ 100 U/mL of penicillin, and 50 ~ 100 $\mu$g/mL of streptomycin. The medium available for the present invention is not limited to the described medium. So long as it allows neuroblast cells to grow well, any medium may be used without limitations.

Turning to a temperature condition, the cells may be cultured at 20 ~ 45 °C, and more preferably at 30 ~ 40°C. For example, during incubation at less than 20°C, the elastin-like extracellular matrix may be dissolved into the medium, thus insufficiently helping 3D neuroblast cell clusters form. On the other hand, at higher than 45°C, the cells cannot grow well nor form 3D neuroblast cell clusters. So long as it has no effects on the elastin-like extracellular matrix and effectively allows for the proliferation of cells and the formation of 3D neuroblast cell clusters, any temperature is available for the present invention, without limitations.

A next consideration is a period of culturing time. It may be preferably set forth to be 12 to 240 hrs, and more preferably 24 to 120 hrs. For example, when the cells are cultured for less than 12 hrs, the neuroblast cell cluster may be small in size and thus low in activity. On the other hand, after culturing for more than 240 hrs, cell death may happen inside the cell cluster.

The atmosphere in which the cells are cultured may contain 3 to 7% $CO_2$ and humidified 93 to 97% air. This atmosphere condition is not limitative, but may be varied so as to facilitate cell proliferation and the formation of 3D neuroblast cell clusters.

Finally, the culture may be a stationary culture, a roller bottle cell culture, an adherent culture, and/or a suspension culture. Preferable is a stationary culture and/or a roller bottle cell culture. So long as it facilitates cell growth and the formation of 3D neuroblast cell clusters, any culture technique may be used without limitations.

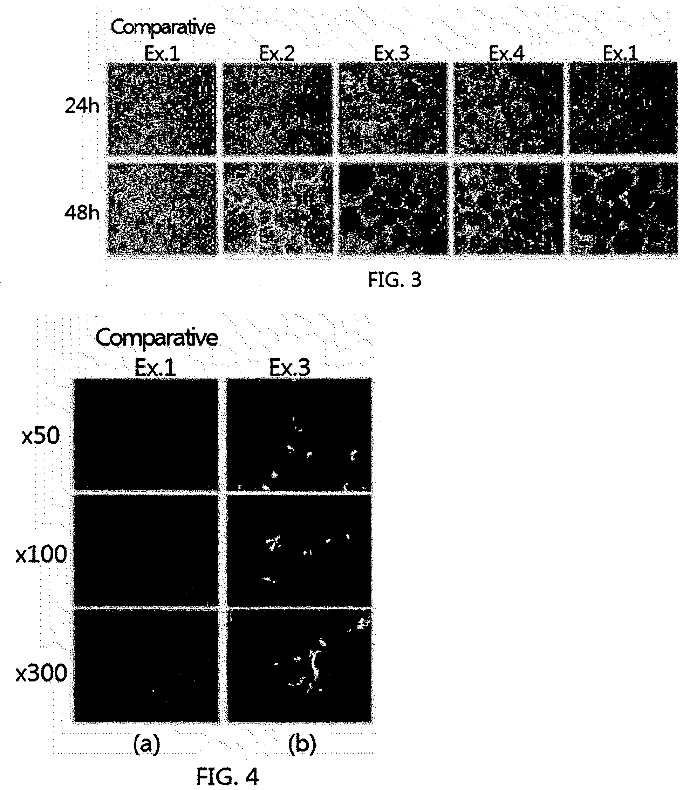

With reference to FIG. 3, there are microphotograph images of neuroblast cells cultured according to one preferred embodiment of the present invention. When cultured in a dish which was not coated with the artificial elastin-like extracellular matrix, neuroblast cells had two-dimensionally spread like a network structure and grown in the form of monolayers, implying that the neuroblast cells strongly adhered to the substrate. In contrast, the neuroblast cells in the artificial elastin-like extracellular matrix-coated culture dishes were found to partially aggregate into clusters within 24 hrs of culture, and finally, 3D neuroblast cell clusters appeared in a spheroid form at 48 h as the aggregation proceeded with time. In addition, the neuroblast cells were observed to aggregate more intensively from earlier phases in cell dishes coated with higher concentrations of the artificial elastin-like extracellular matrix.

FIG. 4 shows SEM images of cells cultured according to a preferred embodiment of the present invention and a comparative embodiment. While cells were two-dimensionally spread across the dish not coated with the artificial elastin-like extracellular matrix as shown in FIG. 4a, cells cultured in dishes coated with the artificial elastin-like extracellular matrix formed 3D spheroidal cell clusters in which individual cells are directly adherent to adjacent ones, as shown in FIG. 4b.

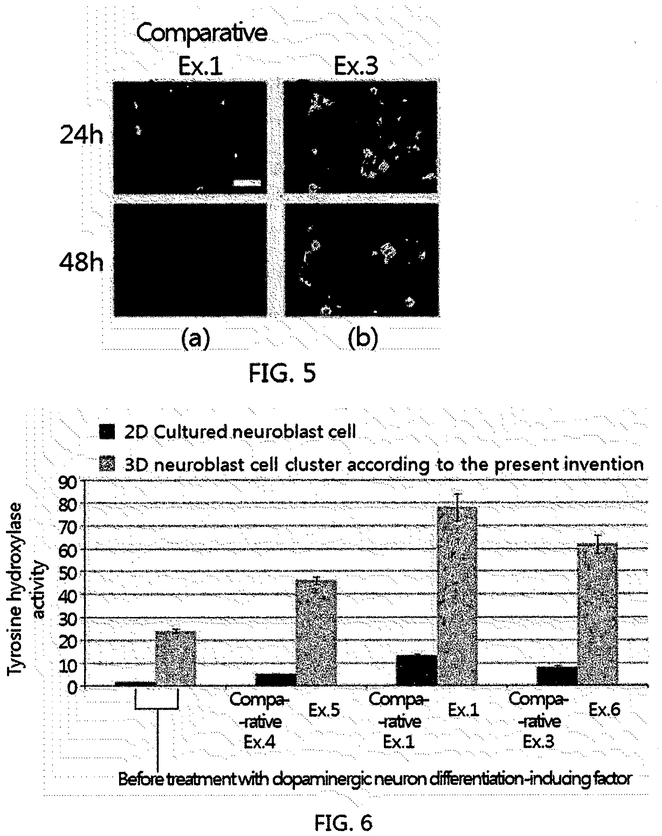

Further, the 3D neuroblast cell cluster may be resistant to the cytotoxicity which may come from the artificial elastin-like extracellular matrix. FIG. 5 shows microphotograph images of 3D neuroblast cell clusters after a viability assay. As shown, only green-stained cells are observed, with no visualization of red fluorescence, even though the cells were cultured for a prolonged period of time. This result indicates that there is no cell death during culture according to the present invention, and thus that the artificial elastin-like extracellular matrix does not exert cytotoxicity on the neuroblast cells cultured thereon.

Preferably, the 3D neuroblast cell clusters are those that have been cultured for 3~5 days, and each may have a diameter of 30 to 200 $\mu$m. A 3D neuroblast cell cluster with a diameter of less than 30 $\mu$m may be unlikely to differentiate into neurons. On the other hand, if it is larger in diameter than 300 $\mu$m, the 3D neuroblast cell cluster may undergo cell death at the inside thereof.

In a subsequent step of the method, a dopaminergic neuron differentiation-inducing factor is added to the 3D neuroblast cell cluster to form a 3D dopaminergic neuron-like cell cluster.

After being cultured in a two- or three-dimensional manner by conventional techniques, neuroblast cells were observed to differentiate into dopaminergic neurons at poor rates even in the presence of a differentiation-inducing factor. Further, an additional process of separating the differentiated dopaminergic neurons from the cell culture is required because of the poor differentiation rate. Korean Patent Application No. 2011-0112406, filed by the present inventor, addresses a method for forming 3-dimensional multicellular spheroids using an artificial elastin-like extracellular matrix, and 3D multicellular spheroids established thereby. In light of the technical level at the time of the application, the 3D multicellular spheroids established by the method were, however, anticipated to poorly differentiate into dopaminergic neurons even in the presence of a dopaminergic neuron differentiation-inducing factor, as in conventional techniques. Therefore, a process of separating dopaminergic neurons from the cell culture was anticipated to be needed additionally, as well.

In contrast, the 3D neuroblast cell cluster according to the present invention is highly apt to differentiate into dopaminergic neurons in response to a dopaminergic neuron differentiation-inducing factor, thereby allowing dopaminergic neuron cells to be obtained from the cultured 3D cell clusters, without an additional isolating process.

For example, 3D multicellular spheroids established using an artificial elastin-like extracellular matrix as disclosed in Korean Patent Application No. 2011-0112406, filed by the present inventor, was induced to differentiate into adult neurons in the presence of retinoic acid, but the differentiation rate is far less than that obtained in the present invention.

With reference to FIG. 6, the 3D dopaminergic neuron-like cell cluster according to one preferred embodiment of the present invention is compared with 2D dopaminergic neuron-like cells in terms of tyrosine hydroxylase (TH) activity. Tyrosine hydroxylase (TH) is an enzyme involved in synthesizing dopamine in dopaminergic neurons, so that it can be used as a marker indicative of differentiation into dopaminergic neurons.

Upon treatment with a differentiation-inducing factor, as can be seen in FIG. 6, a lower activity of tyrosine hydroxylase (TH), a maker for dopaminergic neurons, was detected from two-dimensionally (plane) cultured cells in a typical medium than from 3D neuroblast cell cluster according to the present invention, indicating that the 3D neuroblast cell cluster of the present invention is further induced to differentiate into dopaminergic neurons than the two-dimensionally cultured neuroblast cells by the differentiation-inducing factor because signal transmission and exchange between individual cells within the 3D cell clusters are activated.

In addition, the differentiation rate from neuroblast to neuron of the 3D cell cluster according to the present invention is superior to that of the cells according to conventional 3D culture methods.

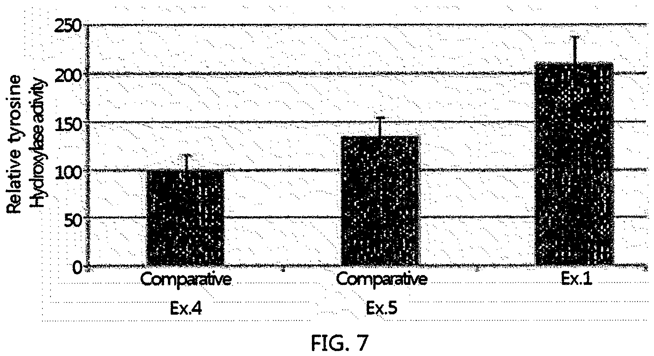

FIG. 7 shows tyrosine hydroxylase activity of the cells differentiated in the presence of phorbol 12-myristate 13-acetate from 3D cells clusters cultured by a conventional method or the method of the present invention, relative to tyrosine hydroxylase activity of the cells of Comparative Example 4. As can be seen from the graph, the cells differentiated from the cell cluster of Example 1 exhibited the enzyme activity 2- and 1.3-fold higher than those differentiated from the cells of Comparative Examples 4 and 5, respectively. The higher the enzyme activity is, the greater the rate of differentiation into dopaminergic neurons is evaluated to be. Thus, the cell cluster of Example 1 can differentiate into dopaminergic neurons at a significantly high rate, compared to Comparative Examples 4 and 5.

In one preferred embodiment, the differentiation-inducing factor may be added in an amount of $1 \times 10^{-7}$ to $1 \times 10^{-2}$ parts by weight, based on 100 parts by weight of the 3D neuroblast cell cluster to induce differentiation into a 3D dopaminergic neuron-like cell cluster. For example, when the differentiation-inducing factor is used in an amount less than $1 \times 10^{-7}$ parts by weight, differentiation into dopaminergic neurons may proceed at a low rate; with some of the 3D cell clusters remaining undifferentiated. On the other hand, the use of the differentiation-inducing factor in an amount greater than $1 \times 10^{-2}$ parts by weight would cause cell death.

For use in the present invention, the differentiation-inducing factor is phorbol 12-myristate 13-acetate.

In accordance with another aspect thereof, the present invention provides a dish for culturing dopaminergic neuron-like cell clusters comprising an artificial elastin-like extracellular matrix coating the dish to improve cell-to-cell interaction, said artificial elastin-like extracellular matrix having the amino acid set forth in the following General Formula 1:

$$[(N-terminus)-TGPG[VGRGD(VGVPG)_{\alpha}]_{\beta}WPC-(C-terminus)]$$

wherein $\alpha$ is an integer meeting $2 \leq \alpha \leq 10$, and $\beta$ is an integer meeting $10 \leq \beta \leq 30$.

A description of the culture dish and the artificial elastin-like extracellular matrix is omitted as it is given in the foregoing.

The 3D dopaminergic neuron-like cell clusters expresses tyrosine hydroxylase (TH), a marker for dopaminergic neurons, and preferably has a TH activity of 45 % or higher, more preferably 60 % or higher, or most preferably 75 % or higher.

Preferably, the 3D dopaminergic neuron-like cell clusters are those that have been cultured for 3-5 days, and each may have a diameter of 30 to 200 $\mu$m. A 3D dopaminergic neuron-like cell cluster with a diameter of less than 30 $\mu$m may be poor in cellular activity. Given a diameter greater than 300 $\mu$m, the 3D dopaminergic neuron-like cell cluster may undergo cell death at the inside thereof.

A better understanding of the present invention may be obtained through the following examples which are set forth to illustrate, but are not to be construed as the limit of the present invention.

EXAMPLE 1

At 4°C, 1 ml of 10 $\mu$M elastin-like extracellular matrix (TGPG[VGRGD(VGVPG)$_6$]$_{20}$WPC) was mixed with 9 ml of PBS to give a coating solution which was then added in an amount of 0.3 ml per well to a 24-well polystyrene cell culture plate (each well having a volume of 1900 $\mu$l, a height of 17.4 mm, and an internal bottom area of 19.1 mm$^2$) and left at 37°C so that the surface of the plate was coated with the matrix. SH-SY5Y cells was seeded at a density of $1 \times 10^5$ cells/well to the coated plate, and wetted with 10% complete medium (a mixture of 1:1: DMEM : Ham's F12 medium, supplemented with 10 vol % fetal bovine serum, 100 U/mL penicillin, and 100 $\mu$g/mL streptomycin, followed by incubation at 37°C for 48 hrs in a humidified 5% $CO_2$ incubator to form 3D neuroblast cell clusters. Then, the 3D neuroblast cell clusters were incubated in the presence of 100 nM of phorbol 12-myristate-13-acetate/$10^5$ cells at 37°C for 48 hrs in a humidified 5% $CO_2$ incubator to yield 3D dopaminergic neuron-like cell clusters.

EXAMPLE 2

Three-dimensional dopaminergic neuron cell clusters were prepared in the same manner as in Example 1, with the exception that 0.1 $\mu$M elastin-like extracellular matrix (TGPG[VGRGD(VGVPG)$_6$]$_{20}$WPC) was used instead of the 10 $\mu$M matrix.

EXAMPLE 3

Three-dimensional dopaminergic neuron cell clusters were prepared in the same manner as in Example 1, with the exception that 1 $\mu$M elastin-like extracellular matrix (TGPG[VGRGD(VGVPG)$_6$]$_{20}$WPC) was used instead of the 10 $\mu$M matrix.

EXAMPLE 4

Three-dimensional dopaminergic neuron cell clusters were prepared in the same manner as in Example 1, with the exception that 5 $\mu$M elastin-like extracellular matrix (TGPG[VGRGD(VGVPG)$_6$]$_{20}$WPC) was used instead of the 10 $\mu$M matrix.

EXAMPLE 5 (not according to the invention)

Three-dimensional dopaminergic neuron cell clusters were prepared in the same manner as in Example 1, with the exception that 100 nM of acidic fibroblast growth factor (FGF-1), instead of 100 nM of Phorbol 12-myristate 13-acetate, was used per $10^5$ cells.

EXAMPLE 6 (not according to the invention)

Three-dimensional dopaminergic neuron cell clusters were prepared in the same manner as in Example 1, with the exception that 100 nM of dibutylcycloAMP, instead of 100 nM of Phorbol 12-myristate 13-acetate, was used per $10^5$ cells.

COMPARATIVE EXAMPLE 1

Three-dimensional dopaminergic neuron cell clusters were prepared in the same manner as in Example 1, with the exception that 0 $\mu$M elastin-like extracellular matrix (TGPG[VGRGD(VGVPG)$_6$]$_{20}$WPC) was used instead of the 10 $\mu$M matrix.

COMPARATIVE EXAMPLE 2

Three-dimensional dopaminergic neuron cell clusters were prepared in the same manner as in Comparative Example 1, with the exception that 100 nM of acidic fibroblast growth factor (FGF-1), instead of 100 nM of Phorbol 12-myristate 13-acetate, was used per $10^5$ cells.

COMPARATIVE EXAMPLE 3

Three-dimensional dopaminergic neuron cell clusters were prepared in the same manner as in Comparative Example 1, with the exception that 100 nM of dibutylcycloAMP, instead of 100 nM of Phorbol 12-myristate 13-acetate, was used per $10^5$ cells.

COMPARATIVE EXAMPLE 4

Three-dimensional dopaminergic neuron cell clusters were prepared in the same manner as in Example 1, with the exception that 10 $\mu$M collagen was used instead of the 10 $\mu$M elastin-like extracellular matrix (TGPG[VGRGD(VGVPG)$_6$]$_{20}$WPC) and that the cells were cultured in a stagnant, roller bottle manner.

COMPARATIVE EXAMPLE 5

Three-dimensional dopaminergic neuron cell clusters were prepared in the same manner as in Example 1, with the exception that 10 $\mu$M collagen was used instead of the 10 $\mu$M elastin-like extracellular matrix

(TGPG[VGRGD(VGVPG)$_6$]$_{20}$WPC) and that the cells were cultured in a suspension manner.

EXPERIMENTAL EXAMPLE 1

The expression of cell adhesion molecules (CAMs) in the cultured neuroblast cells was examined using quantitative real time PCR (qRT-PCR).

In this regard, total RNA was isolated from the neuroblast cells before differentiation into dopaminergic neurons in Example 3 and Comparative Example 1. First, 1 ml of TRIzol reagent (Invitrogen) was added to each sample to homogenize the cells. Then, 200 $\mu$l of chloroform was added to the sample and incubated for 15 min. After centrifugation at 4°C at 12000 rpm for 15 min, the supernatant was recentrifuged at 4°C at 12000 rpm for 10 min, together with 500 $\mu$l of isopropanol, to precipitate RNA. From the RNA, cDNA was synthesized using High-Capacity cDNA reverse transcription kit, Applied Biosystem. Briefly, RNA weighing 4 $\mu$g was mixed with a reverse transcription (RT) buffer, a dNTP mix, RT random primers, MutiScribe Reverse Transcriptase, and nuclease-free H$_2$O, and subjected to thermal reactions at 25°C for 10 min, at 37°C for 120min, and at 85°C for 5 min, sequentially, to synthesize cDNA. The cDNA was stored in 40 $\mu$l of nuclease-free H$_2$O, and per well, 1 $\mu$l of cDNA was mixed with 10 pmole primer sense and antisense, each 0.5 $\mu$l, 10 $\mu$l of 2x SYBR Green PCR master mix, and 8 $\mu$l of nuclease-free H$_2$O, all contained in SYBR Green PCR master mix kit. The PCR reactant thus obtained was subjected to qRT-PCR. It started with heating at 50°C for 2 min and then at 95°C for 10 min, followed by 40 thermal cycles of 95°C for 15 sec and 60°C for 1 min. Primer sets were designed on the basis of human genome sequence using Primer Express 3.0, and are listed in Table 2, below.

TABLE 2

|  | Sense (5'→3') | antisense (5'→3') |
|---|---|---|
| 1-type Collagen | GAGGGCCAAGACGAAGACATC | GGTTGTCGCAGACGCAGAT |
| 4-type Collagen | CGAGCTCTACCGCAACGACTA | AGGCTTCGTGTTTCATGACCTT |
| Laminin $\alpha$4 | GCCTGCGGCCGAGAA | TCCTGAGCCGTCCAAACACT |
| Fibronectin | CCTCGGGCCCAGATAACAG | CGAGGGATACGGTGTACTCAGAT |
| N-cadherin) | CACCATATGATCCCCTGTTAGTGTTT | TCCTGCTCACCACCACTACTTG |
| NCAM2 | CTGCTGCTAATTCTTGTGGTAACAG | CTGGAGCCACTTTTCTTTCCA |
| ICAM1 | GCCGGCCAGCTTATACACA | TTCTGGCCACGTCCAGTTTC |
| Connexin-26 | GTGAACAAACACTCCACCAGCAT | CTCCCCACACCTCCTTTGC |
| GAPDH | ACCCACTCCTCCACCTTTGA | TGTTGCTGTAGCCAAATTCGTT |

Transcription levels of CAMs and ECM materials in the neuroblast cell clusters of Example 1 prior to differentiation into dopaminergic neuron-like cell clusters are depicted, relative to those in the neuroblast cell clusters of Comparative Example 1, in FIGS. 1 and 2.

As can be seen in FIG. 1, the neuroblast cells of Example 1 increased in the mRNA level of CAMs, for example, N-cadherin by 3 folds, neural cell adhesion molecule 2 (NCAM2) by 11 folds, intercellular adhesion molecule-1 (ICAM1) by 17 folds, and connexin-26 by 13 folds, compared to the neuroblast cells of Comparative Example 1.

Also, the mRNA level of ECM in the neuroblast cells of Example 1 was increased by 2 folds for collagen type 1, by 3.7 folds for collagen type 4, by 5.2 folds for laminin $\alpha$4, and by 4 folds for fibronectin, compared to Comparative Example 1, as shown in FIG. 2.

EXPERIMENTAL EXAMPLE 2

Neuroblast cells before differentiation into dopaminergic neurons, prepared in Examples 1 to 4 and Comparative Example 1, were examine by microphotography (Leica DMI 3000B microscope), and their morphologies are shown in FIG. 3.

When cultured in a culture dish which was not coated with the artificial elastin-like extracellular matrix (Comparative Example 1), as shown in FIG. 3, the neuroblast cells spread in a two-dimensional manner, like a network, to form monolayers, implying the predominance of attachment between the neuroblast cells and the dish over between the neuroblast cells themselves. In contrast, the neuroblast cells cultured in the artificial elastin-like extracellular matrix-coated culture dish (Examples 1 to 4) partially aggregated within 24 hrs to form clusters which were developed to 3D spheroids. In addition, the aggregation of the neuroblast cells was denser from the early stage of culture as the concentration of the artificial elastin-like extracellular matrix was higher.

EXPERIMENTAL EXAMPLE 3

Neuroblasts cultured in Examples 3 and Comparative Example 1 were fixed in 2% paraformaldehyde for 15 min at room temperature, and washed twice with phosphate buffered saline (PBS). After being naturally dried, the cells were examined by SEM (Hitachi S-4800 scanning electron microscope). The results are given in FIG. 4.
In FIG. 4a (neuroblast cells cultured in Comparative Example 1), the cells are observed to widely spread while the cells of FIG. 4b (3D neuroblast cell clusters) adhere directly to one another.

EXPERIMENTAL EXAMPLE 4

To examine cell viability, the neuroblast cells cultured in Example 3 and Comparative Example 1 were washed three times with PBS, and stained with a dye containing 2 $\mu$M calcein (AM) and 4 $\mu$M ethidium homodimer-1 (Live/Dead cell staining reagent) at room temperature for 30 min, followed by taking fluorescence images using Leica DMI 3000B microscope. Results are given in FIG. 5.
In FIG. 5b showing the neuroblast cells cultured in Example 3, green fluorescent cells are visualized, but no cells appear with red fluorescence, indicating that the artificial elastin-like extracellular matrix does not exert cytotoxicity on neuroblast cells cultured thereon.

EXPERIMENTAL EXAMPLE 5

Examination was made of differentiation into dopaminergic neurons. Cells cultured in Examples 1, 5 and 6 and Comparative Examples 1 to 6 were washed three times with PBS, and homogenized by a homogenizer (IKA T10, Ultra-Turrax), followed by centrifugation (4°C, 3,000 rpm, 10 min) in a centrifuge (Effendorf centrifuge 5415R). The supernatant thus obtained was used to measure tyrosine hydroxylase activity as follows. The results are given in FIG. 6.

1. Assay for tyrosine hydroxylase activity

Tyrosine hydroxylate activity was assayed according to the process established by Sigma-Aldrich as follows.
With 0.2 ml of each of the proteins from the cells of Examples 1, 5 and 6 and Comparative Examples 1 to 5, 1.5 ml of 100 mM Tris-HCl buffer (pH 7.0), 0.03 ml of 30 mM L-tyrosine (solvent Tris-HCl buffer), 0.05 ml of 10 mM 6,7-dimethyl-5,6,7,8-Tetrahydropterine (DMTHP), 1.0 ml of 0.43 mM $\beta$-NADH ($\beta$-nicotinamide adenine dinucleotide, reduced form, solution), 0.05 ml of catalase, and 0.1 ml of DHT-RDase (dihydropteridine reductase enzyme solution) were mixed together, and the samples were monitored for absorbance at 340 nm at 37°C for 15 min to measure changes of absorbance per min (ABS 340nm/min). From the measurements, TH activity was calculated according to the following equation. For sample blank, 0.2 ml of distilled water was used instead of the proteins. Results of the assay for tyrosine hydroxylase activity are depicted in FIGS. 6 and 7.

$$\text{Tyrosine Hydroxylase Activity} = (\text{A}_{340nm}/\text{min Test} - \text{A}_{340nm}/\text{min Blank}) \times 2.93 \times 1000 / 0.19$$

FIG. 6 is a graph of the tyrosine hydroxylase activity in cells differentiated from the 2D cultured neuroblast cells and the 3D neuroblast cell cluster of the present invention after treatment with dopaminergic neuron differentiation-inducing factor. As can be seen in the graph, both the 2D cells and 3D cell clusters increased in enzyme activity after exposure to dopaminergic differentiation-inducing factor; but with significantly higher levels detected from the 3D cell clusters of Examples 1, 5 and 6 than those of the 2D cells of Comparative Examples 1 to 3. Considering the fact that a higher level of tyrosine hydroxylase reflects a higher rate of differentiation into dopamine neurons, the neuroblast cells of Examples 1, 5 and 7 is far superior in terms of dopaminergic neuron differentiation rate to those of Comparative Examples 1 to 3. Among them, the highest differentiation rate was measured in the cells of Example 1 upon exposure to phorbol 12-myristate 13-acetate.
FIG. 7 is a graph showing tyrosine hydroxylase activity of the cells differentiated in the presence of phorbol 12-myristate 13-acetate from 3D cells clusters cultured by a conventional method or the method of the present invention, relative to the tyrosine hydroxylase activity of the cells of Comparative Example 4. As can be seen from the graph, the cells differentiated from the cell cluster of Example 1 exhibited the enzyme activity 2- and 1.3-fold higher than those differentiated from the cells of Comparative Examples 4 and 5, respectively. The higher the enzyme activity is, the greater the rate of differentiation into dopaminergic neurons is evaluated to be. Thus, the cell cluster of Example 1 can differentiate into dopaminergic neurons at a significantly high rate, compared to Comparative Examples 4 and 5.

**Claims**

1.  A method for preparing a dopaminergic neuron-like cell cluster, comprising:

    coating a cell culture dish with an artificial elastin-like extracellular matrix having the amino acid set forth in the following General Formula 1:

$$[(\text{N-terminus})\text{-TGPG}[\text{VGRGD}(\text{VGVPG})_\alpha]_\beta\text{WPC-(C-terminus)}]$$

    wherein $\alpha$ is an integer meeting $2 \leq \alpha \leq 10$, and $\beta$ is an integer meeting $10 \leq \beta \leq 30$;
    culturing neuroblast cells in the coated cell culture dish to form a 3D neuroblast cell cluster; and
    adding a dopaminergic neuron differentiation-inducing factor to the 3D neuroblast cell cluster to form a 3D dopaminergic neuron-like cell cluster, wherein the dopaminergic neuron differentiation-inducing factor is phorbol 12-myristate 13-acetate.

2.  The method of claim 1, wherein the coating is carried out by applying the artificial elastin-like extracellular matrix in an amount of 0.05 to 100 $\mu$mol per surface area ($mm^2$) of the culture dish.

3.  The method of claim 1, wherein the neuroblast is selected from the group consisting of: N2a, SH-SY5Y, and a combination thereof.

4.  The method of claim 1, wherein the adding is carried out by using the differentiation-inducing factor in an amount of $1 \times 10^{-7}$ to $1 \times 10^{-2}$ parts by weight, based on 100 parts by weight of the 3D neuroblast cell cluster.

5.  A dish for culturing dopaminergic neuron-like cell clusters comprising:

    an artificial elastin-like extracellular matrix coating the dish to enhance intercellular interaction, the artificial elastin-like extracellular matrix having the amino acid set forth in the following General Formula 1:

$$[(\text{N-terminus})\text{-TGPG}[\text{VGRGD}(\text{VGVPG})_\alpha]_\beta\text{WPC-(C-terminus)}]$$

    wherein $\alpha$ is an integer meeting $2 \leq \alpha \leq 10$, and $\beta$ is an integer meeting $10 \leq \beta \leq 30$; and
    phorbol 12-myristate 13-acetate as a dopaminergic neuron differentiation-inducing factor.

**Patentansprüche**

1.  Verfahren zur Herstellung eines dopaminerge Neuronen-ähnlichen Zellclusters, umfassend:

    Überziehen einer Zellkulturschale mit einer künstlichen elastinähnlichen extrazellulären Matrix, die die Aminosäure, welche in der folgenden allgemeinen Formel 1 dargelegt ist, aufweist:

$$[(\text{N-Terminus})\text{-TGPG}[\text{VGRGD}(\text{VGVPG})_\alpha]_\beta\text{WPC-(C-Terminus)}],$$

    wobei $\alpha$ eine ganze Zahl, die $2 \leq \alpha \leq 10$ erfüllt, ist und $\beta$ eine ganze Zahl, die $10 \leq \beta \leq 30$ erfüllt, ist;
    Kultivieren von Neuroblastenzellen in der überzogenen Zellkulturschale, wobei ein 3D-Neuroblasten-Zellcluster gebildet wird; und
    Zugeben eines dopaminerge Neuronen-Differenzierung-induzierenden Faktors zu dem 3D-Neuroblasten-Zellcluster, um einen dopaminerge Neuronen-ähnlichen 3D-Zellcluster zu bilden, wobei der dopaminerge Neuronen-Differenzierung-induzierende Faktor Phorbol-12-myristat-13-acetat ist.

**2.** Verfahren nach Anspruch 1, wobei das Überziehen durch Verwenden der künstlichen elastinähnlichen extrazellulären Matrix in einer Menge von 0,05 bis 100 µmol pro Flächeninhalt (mm$^2$) der Kulturschale durchgeführt wird.

**3.** Verfahren nach Anspruch 1, wobei der Neuroblast aus der Gruppe ausgewählt sein kann, welche besteht aus: N2a, SH-SY5Y und einer Kombination davon.

**4.** Verfahren nach Anspruch 1, wobei das Zugeben durch Verwenden des Differenzierung-induzierenden Faktors in einer Menge von 1 x 10$^{-7}$ bis 1 x 10$^{-2}$ Gewichtsteilen, bezogen auf 100 Gewichtsteile des 3D-Neuroblasten-Zellclusters, durchgeführt wird.

**5.** Schale zum Kultivieren von dopaminerge Neuronen-ähnlichen Zellclustern, umfassend:

eine künstliche elastinähnliche extrazelluläre Matrix, die die Schale überzieht, um interzelluläre Wechselwirkung zu steigern, wobei die künstliche elastinähnliche extrazelluläre Matrix die Aminosäure, welche in der folgenden allgemeinen Formel 1 dargelegt ist, aufweist:

$$[(\text{N-Terminus})\text{-TGPG}[\text{VGRGD}(\text{VGVPG})_\alpha]_\beta\text{WPC-(C-Terminus)}],$$

wobei $\alpha$ eine ganze Zahl, die $2 \leq \alpha \leq 10$ erfüllt, ist und $\beta$ eine ganze Zahl, die $10 \leq \beta \leq 30$ erfüllt, ist; und Phorbol-12-myristat-13-acetat als einen dopaminerge Neuronen-Differenzierung-induzierenden Faktor.

**Revendications**

**1.** Procédé de préparation d'un agrégat de cellules de type neurones dopaminergiques, comprenant :

un revêtement d'une boîte de culture cellulaire d'une matrice extracellulaire artificielle de type élastine ayant l'acide aminé défini par la formule générale 1 suivante :

$$[(\text{N-terminal})\text{-TGPG}[\text{VGRGD}(\text{VPVPG})_\alpha]_\beta\text{WPC-(C-terminal)}]$$

dans lequel $\alpha$ est un entier satisfaisant $2 \leq \alpha \leq 10$ et $\beta$ est un entier satisfaisant $10 \leq \beta \leq 30$ ;
une culture des cellules neuroblastes dans la boîte de culture cellulaire revêtue pour former un agrégat 3D de cellules neuroblastes ; et
un ajout d'un facteur d'induction de différenciation des neurones dopaminergiques à l'agrégat 3D de cellules neuroblastes pour former un agrégat 3D de cellules de type neurones dopaminergiques,

dans lequel le facteur d'induction de différenciation des neurones dopaminergiques est le phorbol 12-myristate 13-acétate.

**2.** Procédé selon la revendication 1, dans lequel le revêtement est réalisée en appliquant une quantité de matrice extracellulaire artificielle de type élastine de 0,05 à 100 µmol par unité de surface (mm$^2$) de la boîte de culture.

**3.** Procédé selon la revendication 1, dans lequel le neuroblaste est choisi dans le groupe constitué de : N2a, SH-SY5Y, et d'une combinaison de ceux-ci.

**4.** Procédé selon la revendication 1, dans lequel l'ajout est réalisé en utilisant une quantité du facteur d'induction de différenciation de 1x10$^{-7}$ à 1x10$^{-2}$ parties en poids, pour 100 parties en poids de l'agrégat 3D de cellules neuroblastes.

**5.** Boîte de culture d'agrégats de cellules de type neurones dopaminergiques comprenant :

une matrice extracellulaire artificielle de type élastine revêtant la boîte pour améliorer l'interaction intercellulaire, la matrice extracellulaire artificielle de type élastine ayant l'acide aminé défini par la formule générale 1 suivante :

$$[(N\text{-terminal})\text{-TGPG}[\text{VGRGD}(\text{VPVPG})_\alpha]_\beta\text{WPC-}(C\text{-terminal})]$$

dans lequel $\alpha$ est un entier satisfaisant $2 \leq \alpha \leq 10$, et $\beta$ est un entier satisfaisant $10 \leq \beta \leq 30$ ; et du phorbol 12-myristate 13-acétate en tant que facteur d'induction de différenciation des neurones dopaminergiques.

FIG. 1

FIG. 2

FIG. 3

FIG. 4

FIG. 5

FIG. 6

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 20110112406
- KR 20130047404 A

### Non-patent literature cited in the description

- **GWON-SOO JUNG et al.** Morphogenetic and neuronal characterization of human neuroblastoma multicellular spheroids cultured under undifferentiated and all-trans-retinoic acid-differentiated conditions. *BMB Reports,* 31 May 2013, vol. 46 (5), 276-281
- **S. SHAVALI et al.** Synergistic neurotoxic effects of arsenic and dopamine in human dopaminergic neuroblastoma SH-SY5Y Cells. *Toxicological Sciences,* 18 October 2007, vol. 102 (2), 254-261
- **WON BAE JEON et al.** Functional enhancement of neuronal cell behaviors and differentiation by elastin-mimetic recombinant protein presenting Arg-Gly-Asp peptides. *BMC Biotechnology,* 14 September 2012, vol. 12 (1), 61
- **GILDA RAGUENEZ et al.** BCL-2 is upregulated in human SH-SY5Y neuroblastoma cells differentiated by overexpression of fibrolast growth factor 1. *Biochemical and biophysical research communications,* 01 May 1999, vol. 258 (3), 745-751
- **ELENA DI DANIEL et al.** Comparative analysis of the effects of four mood stabilizers in SH-SY5Y cells and in primary neurons. *Bipolar Disorders,* 01 February 2005, vol. 7 (1), 33-41
- **KUME et al.** Dibutyryl cyclic AMP induces differentiation of human neuroblastoma SH-SY5Y cells into a noradrenergic phenotype. *Neuroscience Letters,* 10 October 2008, vol. 443 (3), 199-203